(19) Europäisches Patentamt
European Patent Office
Office européen des brevets

(11) Numéro de publication: **0 256 936 A1**

(12) # DEMANDE DE BREVET EUROPEEN

(21) Numéro de dépôt: 87401834.4

(22) Date de dépôt: 07.08.87

(51) Int.Cl.[3]: C 07 D 295/02
C 07 D 213/74, C 07 D 471/04
C 07 D 487/04, A 61 K 31/495
//(C07D471/04,221:00,209:00),
(C07D487/04,241:00,209:00),
(C07D487/04,239:00,209:00)

(30) Priorité: 12.08.86 FR 8611617

(43) Date de publication de la demande:
24.02.88 Bulletin 88/8

(84) Etats contractants désignés:
AT BE CH DE ES FR GB GR IT LI LU NL SE

(71) Demandeur: RHONE-POULENC SANTE
Les Miroirs 18 Avenue d'Alsace
F-92400 Courbevoie Cédex(FR)

(72) Inventeur: Barreau, Michel
24 bis avenue du Clos de Sénart
F-91230 Montgeron(FR)

(72) Inventeur: Comte, Marie-Thérèse
1 Allée Costes et Bellonte
F-94550 Chevilly Larue(FR)

(72) Inventeur: Malleron, Jean-Luc
5 Allée Véronèse Résidence l'Ermitage
F-91940 Les Ulis(FR)

(72) Inventeur: Ponsinet, Gérard
7 rue de Grand Champ
F-94370 Sucy en Brie(FR)

(74) Mandataire: Gaumont, Robert et al,
RHONE-POULENC INTERSERVICES Brevets Pharma 25,
Quai Paul Doumer
F-92408 Courbevoie Cédex(FR)

(54) **Nouveaux dérivés de la benzyl-4 pipérazine, leur préparation et les compositions pharmaceutiques qui les contiennent.**

(57) Nouveaux produits de formule (I) dans laquelle la ligne pointillée forme un cycle phényle, naphtyle, pyridyle, indolizine, pyrrolo[1,2-a] pyrazine, pyrrolo[1,2-a] pyrimidine ou pyrrolo[1,2-c] pyrimidine, R = H, halogène, OH, alcoyle, alcoyloxy ou alcoylthio, R' = H, halogène, alcoyle, alcoyloxy, alcoylthio, CN ou $CF_3$, n et p = 1, 2 ou 3, les radicaux alcoyle ayant 1 à 4 C en chaîne droite ou ramifiée.

Ces produits sont utiles comme neuroleptiques.

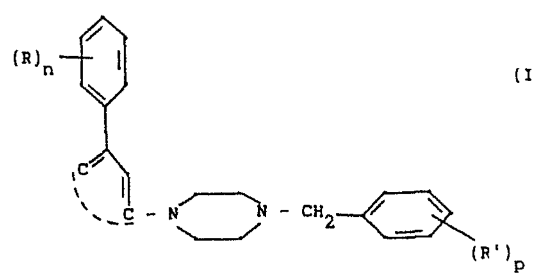

(I)

EP 0 256 936 A1

La présente invention concerne de nouveaux dérivés de la benzyl-4 pipérazine de formule générale :

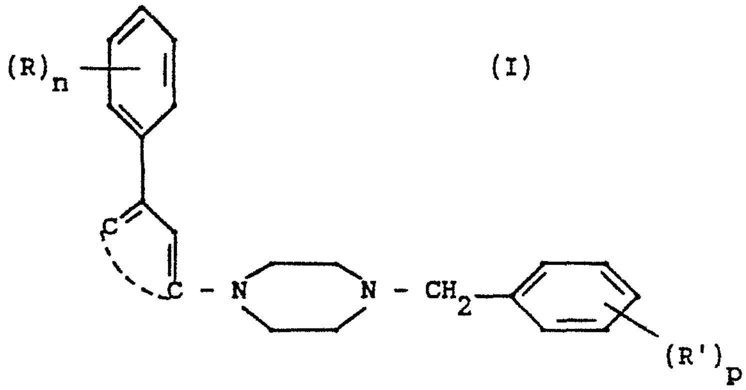

(I)

dans laquelle la ligne pointillée forme avec la chaîne de quatre atomes de carbone à laquelle elle se rattache un cycle aromatique choisi parmi les cycles phényle, naphtyle, pyridine, indolizine, pyrrolo[1,2-a] pyrazine, pyrrolo[1,2-a] pyrimidine ou pyrrolo[1,2-c] pyrimidine, R représente un atome d'hydrogène ou d'halogène ou un radical hydroxy, alcoyle, alcoyloxy ou alcoylthio, R' représente un atome d'hydrogène ou d'halogène ou un radical alcoyle, alcoyloxy, alcoylthio, cyano ou trifluorométhyle, n et p représentent les nombres entiers 1, 2 ou 3, étant entendu que les radicaux alcoyle et portions alcoyle contiennent 1 à 4 atomes de carbone en chaîne droite ou ramifiée, ainsi que leurs sels d'addition.

Selon l'invention, les produits de formule générale (I) dans laquelle la ligne pointillée forme avec la chaîne de quatre atomes de carbone à laquelle elle se rattache un cycle phényle ou naphtyle peuvent être préparés par action d'un dérivé de formule générale :

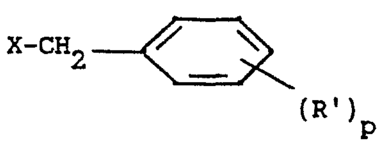

(II)

dans laquelle R' et p sont définis comme précédemment et X représente un atome d'halogène tel que le chlore ou le brome ou un reste d'ester activé tel que le radical mésyloxy ou tosyloxy, sur un produit de formule générale :

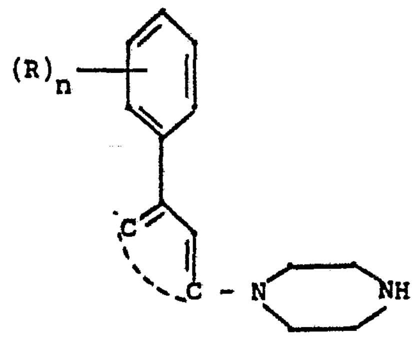

(III)

dans laquelle la ligne pointillée forme avec la chaîne de quatre atomes de carbone à laquelle elle se rattache un cycle phényle ou naphtyle et les autres symboles sont définis comme précédemment.

On opère généralement dans un solvant organique tel que l'acétonitrile ou un solvant chloré tel que le chloroforme en présence d'un accepteur d'acide tel qu'un carbonate comme le carbonate de potassium ou la diméthylamino-4 pyridine, à une température comprise entre 20°C et la température de reflux du mélange réactionnel.

Les produits de formule générale (III) peuvent être préparés par action de la bis-(chloro-2 éthyl) amine sur une amine de formule générale :

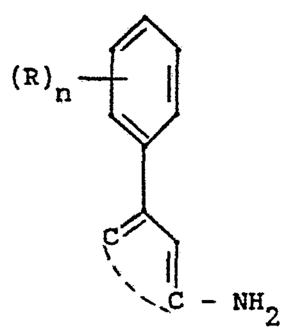

(IV)

dans laquelle la ligne pointillée forme avec la chaîne de quatre atome de carbone à laquelle elle se rattache un cycle phényle ou naphtyle et les autres symboles sont définis comme précédemment.

On opère généralement dans un solvant à haut point d'ébullition tel que le trichloro-1,2,4 benzène à une température comprise entre 150°C et la température de reflux du mélange réactionnel.

Les produits de formule générale (IV) peuvent être préparés par application ou adaptation des méthodes décrites dans la littérature.

Selon l'invention, les produits de formule générale (I) dans laquelle la ligne pointillée forme avec la chaîne de quatre atomes de carbone à laquelle elle se rattache un cycle pyridine peuvent être préparés par action d'une pipérazine de formule générale :

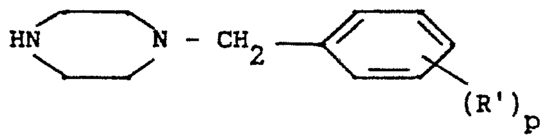

(V)

dans laquelle R' et p sont définis comme précédemment sur un produit de formule générale :

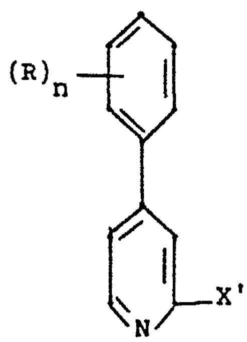

(VI)

dans laquelle R et n sont définis comme précédemment et X' représente un atome d'halogène tel que le chlore ou le brome.

On opère généralement dans un solvant organique à haut point d'ébullition tel que le trichloro-1,2,4 benzène à une température comprise entre 80°C et la température de reflux du mélange réactionnel.

Les produits de formule générale (VI) peuvent être préparés par adaptation des méthodes connues dans la littérature pour obtenir une halogéno-2 pyridine, par exemple par action du chlorure de phosphoryle sur un produit de formule générale :

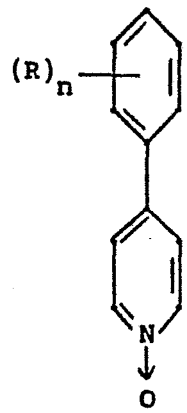

(VII)

dans laquelle R et n sont définis comme précédemment.

On opère généralement par chauffage en autoclave à une température voisine de 190°C.

Les produits de formule générale (VII) peuvent être obtenus par application ou adaptation des méthodes connues et décrites dans la littérature.

Selon l'invention, les produits de formule générale (I) dans laquelle la ligne pointillée forme avec la chaîne de quatre atomes de carbone à laquelle elle se rattache un cycle indolizine, pyrrolo[1,2-a] pyrazine, pyrrolo[1,2-a] pyrimidine ou pyrrolo[1,2-c] pyrimidine peuvent être préparés par action d'une pipérazine de formule générale (V) définie comme précédemment sur un aldéhyde de formule générale :

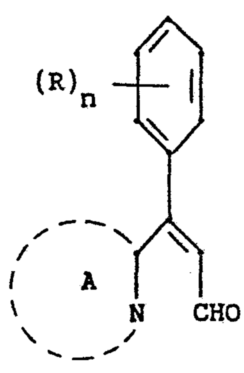

(VIII)

dans laquelle R et n sont définis comme précédemment et A représente un radical pyridyle-2, pyrazinyle, pyrimidinyle-2 ou pyrimidinyle-4.

On opère généralement dans un solvant organique tel qu'un éther comme le tétrahydrofuranne ou le dioxane à une température

comprise entre 50°C et la température de reflux du mélange réactionnel.

Les produits de formule générale (VIII) dans laquelle A représente un radical pyridyle-2 ou pyrazinyle peuvent être préparés par action du (cyclohexylamino-2 vinyl)-1 phosphonate de diéthyle sur un produit de formule générale :

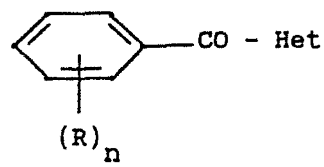

(IX)

dans laquelle R et n sont définis comme précédemment et Het représente un radical pyridyle-2 ou pyrazinyle.

On opère généralement en présence d'une base telle qu'un hydrure comme l'hydrure de sodium dans un solvant organique tel qu'un éther comme le tétrahydrofuranne, à une température comprise entre 25°C et la température de reflux du mélange réactionnel.

Les produits de formule générale (IX) peuvent être préparés par application ou adaptation des méthodes connues.

Les produits de formule générale (VIII) dans laquelle A représente un radical pyrimidinyle peuvent être préparés par oxydation de l'alcool correspondant de formule générale :

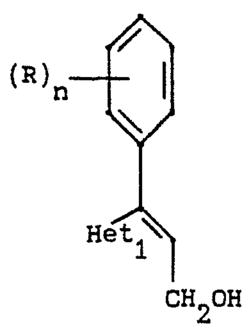

(X)

dans laquelle R et n sont définis comme précédemment et $Het_1$ représente un radical pyrimidinyle-2 ou 4.

On opère généralement par tout moyen connu pour oxyder un alcool en aldéhyde sans affecter le reste de la molécule, par exemple à l'aide de bioxyde de manganèse dans un solvant tel que le dioxane à une température voisine de 20°C.

Les alcools de formule générale (X) peuvent être préparés par réduction des esters de formule générale :

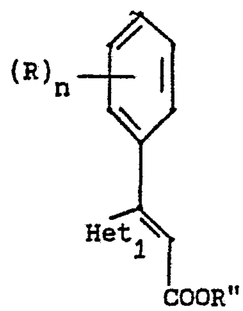

(XI)

dans laquelle $Het_1$ représente un radical pyrimidinyle-2 ou 4, R" représente un radical alcoyle en chaîne droite ou ramifiée contenant 1 à 4 atomes de carbone et R et n sont définis comme précédemment.

On opère par toute méthode connue pour réduire un ester en alcool par exemple au moyen de l'hydrure de diisobutylaluminium dans un solvant comme le toluène à une température voisine de -78°C.

Les esters de formule générale (XI) peuvent être préparés par adaptation de la méthode décrite par E.C. TAYLOR et S.F. MARTIN, J. Am. Chem. Soc. 96, 8095 (1974).

Les nouveaux produits de formule générale (I) peuvent être purifiés par les méthodes connues habituelles, par exemple par cristallisation, chromatographie, extractions successives en milieu acide et basique ou formation de sels et recristallisation de ceux-ci.

Les nouveaux produits de formule générale (I) peuvent être transformés en sels d'addition avec les acides par action d'un acide dans un solvant organique tel qu'un alcool, une cétone, un éther ou un solvant chloré. Le sel formé précipite, éventuellement après concentration de sa solution ; il est séparé par filtration ou décantation.

Les nouveaux produits de formule générale (I) et leurs sels pharmaceutiquement acceptables présentent d'intéressantes propriétés pharmacologiques les rendant utiles comme antipsychotiques. Ils se sont montrés actifs chez la souris à des doses comprises entre 0,5 et 20 mg/kg par voie orale dans le test d'antagonisme des redressements

induits par l'apomorphine selon la technique de P. PROTAIS et coll., Psychopharmacology, 50, 1 (1976).

Les nouveaux produits de formule générale (I) présentent une toxicité faible. Leur $DL_{50}$ est comprise entre 100 et 900 mg/kg par voie orale chez la souris.

Sont particulièrement intéressants les produits de formule générale (I) dans laquelle la ligne pointillée forme avec la chaîne de quatre atomes de carbone à laquelle elle se rattache un cycle aromatique choisi parmi les cycles phényle, naphtyle, pyridine, indolizine, pyrrolo[1,2-a] pyrazine ou pyrrolo[1,2-a] pyrimidine, R représente un atome d'hydrogène, R' représente un atome d'hydrogène ou un radical alcoyle, n et p représentent le nombre entier 1, étant entendu que les radicaux alcoyle et portions alcoyles contiennent 1 à 4 atomes de carbone en chaîne droite ou ramifiée, ainsi que ses sels d'addition avec les acides pharmaceutiquement acceptables.

Sont plus particulièrement intéressants les produits suivants :

- [(méthyl-4 benzyl)-4 pipérazinyl-1]-3 biphényle
- [(méthyl-4 benzyl)-4 pipérazinyl-1]-1 phényl-3 naphtalène
- [(méthyl-4 benzyl)-4 pipérazinyl-1]-2 phényl-4 pyridine
- [(méthyl-4 benzyl)-4 pipérazinyl-1]-3 phényl-1 indolizine
- [(méthyl-4 benzyl)-4 pipérazinyl-1]-3 phényl-1 pyrrolo[1,2-a] pyrazine
- [(méthyl-4 benzyl)-4 pipérazinyl-1]-3 phényl-1 pyrrolo[1,2-a] pyrimidine.

Comme exemples de sels pharmaceutiquement acceptables peuvent être cités les sels d'addition avec les acides minéraux (tels que chlorhydrates, sulfates, nitrates, phosphates) ou organiques (tels que les acétates, propionates, succinates, benzoates, fumarates, maléates, méthanesulfonates, iséthionates, théophylline-acétates, salicylates, phénolphtalinates, méthylène-bis-β-oxynaphtoates) ou des dérivés de substitution de ces composés.

Les exemples suivants, donnés à titre non limitatif, montrent comment l'invention peut être mise en pratique.

EXEMPLE 1

A une solution de 11,9 g de [pipérazinyl-1]-3 biphényle dans 200 cm3 d'acétonitrile, on ajoute 6,9 g de carbonate de potassium puis, en 20 minutes, une solution de 8 g d'α-chloro p-xylène dans 50 cm3 d'acétonitrile, à une température voisine de 20°C et poursuit l'agitation pendant 16 heures. L'insoluble qui s'est formé est séparé par filtration. La solution obtenue est concentrée sous pression réduite (20 mm de mercure ; 2,7 kPa) à 40°C. Le résidu est dissous dans 30 cm3 de chlorure de méthylène et la solution est versée sur 1 kg de silice contenu dans une colonne de 8 cm de diamètre. On élue avec 3 litres d'un mélange de chlorure de méthylène et de méthanol (97,5-2,5 en volumes) et l'éluat correspondant est concentré à sec sous pression réduite (20 mm de mercure ; 2,7 kPa) à 50°C. Le résidu obtenu est dissous dans 600 cm3 d'acétone. On refroidit la solution à une température voisine de 5°C, ajoute 40 cm3 d'éther chlorhydrique 2,5N et maintient l'agitation pendant environ 1 heure. Le précipité formé est séparé par filtration, lavé avec 2 fois 40 cm3 d'acétone. On obtient ainsi 17,3 g de dichlorhydrate de [(méthyl-4 benzyl)-4 pipérazinyl-1]-3 biphényle fondant à 241°C.

Le (pipérazinyl-1)-3 biphényle peut être préparé de la manière suivante : Une solution de 24,3 g d'amino-3 biphényle et de 29,8 g de bis-(chloro-2 éthyl) amine dans 60 cm3 de trichloro-1,2,4 benzène est chauffée pendant 6 heures à 170°C. Après refroidissement à une température voisine de 20°C, on ajoute 400 cm3 d'oxyde d'isopropyle. Le précipité formé est séparé par filtration, lavé avec 3 fois 50 cm3 d'oxyde d'isopropyle puis dissous dans 500 cm3 d'acétate d'éthyle. On ajoute 44 cm3 de solution aqueuse de soude 5N puis 200 cm3 d'eau distillée. La phase organique est décantée et la phase aqueuse est lavée avec 2 fois 100 cm3 d'acétate d'éthyle. Les extraits organiques sont réunis, séchés sur sulfate de magnésium anhydre, filtrés et concentrés à sec sous pression réduite (20 mm de mercure ; 2,7 kPa) à 40°C. Le résidu obtenu est dissous dans 30 cm3 de chlorure de méthylène et la solution est versée sur 1 kg de silice contenu dans une colonne de 8 cm de diamètre. On élue avec 3 litres

d'un mélange de chlorure de méthylène et de méthanol (90-10 en volumes) et l'éluat correspondant est concentré à sec sous pression réduite (20 mm de mercure ; 2,7 kPa) à 50°C. On obtient ainsi 18,8 g de (pipérazinyl-1)-3 biphényle sous forme d'un solide pâteux jaunâtre utilisé sans autre purification dans les synthèses ultérieures.

L'amino-3 biphényle peut être préparé selon la méthode décrite par H.H. BOSSHARD et H. ZOLLINGER, Helv. Chim. Acta 44, 1985 (1961).

EXEMPLE 2

A une solution agitée de 17,9 g de phényl-3 (pipérazinyl-1)-1 naphtalène dans 280 cm3 d'acétonitrile, on ajoute 8,6 g de carbonate de potassium puis, en 20 minutes, une solution de 8,7 g d'α-chloro p-xylène dans 50 cm3 d'acétonitrile, à une température voisine de 20°C et on poursuit l'agitation pendant 16 heures. L'insoluble qui s'est formé est séparé par filtration. La solution obtenue est évaporée sous pression réduite (20 mm de mercure ; 2,7 kPa) à 40°C. Le résidu est dissous dans 35 cm3 de chlorure de méthylène et la solution est versée sur 1 kg de silice contenu dans une colonne de 7,5 cm de diamètre. On élue avec 3 litres d'un mélange de chlorure de méthylène et de méthanol (99-1 en volumes) et l'éluat correspondant est concentré à sec sous pression réduite (20 mm de mercure ; 2,7 kPa) à 50°C. Le résidu obtenu est dissous dans 250 cm3 d'acétone. On refroidit la solution à une température voisine de 5°C, ajoute 52 cm3 de solution 2,5N d'acide chlorhydrique anhydre dans l'éther éthylique et maintient l'agitation pendant environ 4 heures. Le précipité formé est séparé par filtration, lavé avec 3 fois 25 cm3 d'éther éthylique. On obtient ainsi 19 g de dichlorhydrate de [(méthyl-4 benzyl)-4 pipérazinyl-1]-1 phényl-3 naphtalène fondant à 252°C.

Le phényl-3 (pipérazinyl-1)-1 naphtalène peut être préparé de la manière suivante : A une solution de 21,5 g d'amino-1 phényl-3 naphtalène dans 100 cm3 de méthanol, on ajoute 43,3 g de bis-(chloro-2 éthyl) amine en solution dans 100 cm3 de méthanol. Le

mélange est concentré à sec sous pression réduite (20 mm de mercure ; 2,7 kPa) à 50°C. On ajoute au résidu obtenu 200 cm3 de trichloro-1,2,4 benzène et agite à 170°C pendant 13 heures. Après refroidissement à une température voisine de 20°C, on ajoute 300 cm3 d'oxyde d'isopropyle. Le précipité formé est séparé par filtration, lavé avec 3 fois 50 cm3 d'oxyde d'isopropyle puis dissous dans 5 litres d'acétate d'éthyle. On ajoute 200 cm3 d'une solution aqueuse de soude 5N puis 500 cm3 d'eau distillée. La solution organique est décantée et la phase aqueuse est lavée avec 1 litre d'acétate d'éthyle. Les extraits organiques sont réunis, séchés sur sulfate de magnésium anhydre, filtrés et concentrés à sec sous pression réduite (20 mm de mercure ; 2,7 kPa) à 40°C. L'huile obtenue est dissoute dans 40 cm3 de chlorure de méthylène et la solution obtenue est versée sur 600 g de silice contenus dans une colonne de 7 cm de diamètre. On élue avec 3,5 litres d'un mélange de chlorure de méthylène et de méthanol (90-10 en volumes) et l'éluat correspondant est concentré à sec sous pression réduite (20 mm de mercure ; 2,7 kPa) à 50°C. On obtient ainsi 16,9 g de phényl-3 (pipérazinyl-1)-1 naphtalène sous forme d'une huile brune très visqueuse utilisée sans autre purification dans les synthèses ultérieures.

L'amino-1 phényl-3 naphtalène peut être préparé de la façon suivante : Dans une solution de 51,2 g de phényl-3 tétralone-1-oxime, 215 cm3 d'acide acétique et 43 cm3 d'anhydride acétique, on fait barboter pendant 2 heures à une température voisine de 25°C un courant d'acide chlorhydrique gazeux pendant 2 heures et on maintient l'agitation pendant 16 heures. Le précipité formé est séparé par filtration, lavé 3 fois avec 90 cm3 au total d'éther éthylique puis repris par 350 cm3 de chlorure de méthylène. On ajoute au mélange 66 cm3 de solution aqueuse de soude 5N puis 100 cm3 d'eau distillée. La phase organique est décantée et la phase aqueuse est lavée avec 100 cm3 de chlorure de méthylène. Les extraits organiques sont réunis, séchés sur sulfate de magnésium anhydre, filtrés et concentrés à sec sous pression réduite (20 mm de mercure ; 2,7 kPa) à 40°C. Le résidu obtenu est dissous dans 40 cm3 de chlorure de méthylène et la solution est versée sur 1 kg de silice contenu dans une colonne de

7 cm de diamètre. On élue avec 3,5 litres de chlorure de méthylène et l'éluat correspondant est concentré à sec sous pression réduite (20 mm de mercure ; 2,7 kPa) à 40°C. On obtient ainsi 12 g d'amino-1 phényl-3 naphtalène fondant environ à 103°C.

La phényl-3 tétralone-1-oxime peut être préparée de la manière suivante : Une solution de 160 g de phényl-3 tétralone-1 et de 98 g de chlorure d'hydroxylammonium dans 750 cm3 de pyridine et 1500 cm3 d'éthanol est chauffée pendant 3 heures à une température voisine de 80°C. Après refroidissement à une température voisine de 20°C, la solution est concentrée à sec sous pression réduite (20 mm de mercure ; 2,7 kPa) à 60°C. Le résidu est repris par 1,5 litre d'eau distillée et le mélange est chauffé pendant 1 heure à une température voisine de 80°C. Après refroidissement à 20°C le précipité formé est filtré et lavé avec 2 fois 100 cm3 d'eau distillée. On obtient ainsi 85,6 g de phényl-3 tétralone-1-oxime fondant à 195°C.

La phényl-3 tétralone-1 peut être préparée selon la méthode décrite par J.P. QUILLET, A. DUPERRIER et J. DREUX, Bull. Soc. Chim. France. 1, 255 (1967).

EXEMPLE 3

Une solution de 11,4 g de chloro-2 phényl-4 pyridine et de 11,4 g de (méthyl-4 benzyl)-4 pipérazine dans 40 cm3 de trichloro-1,2,4 benzène est chauffée pendant 5 heures à 170°C. Après refroidissement à une température voisine de 20°C, on ajoute 200 cm3 d'oxyde d'isopropyle. Le précipité formé est séparé par filtration, lavé avec 50 cm3 d'oxyde d'isopropyle puis dissous dans 250 cm3 d'acétate d'éthyle. On ajoute 16 cm3 d'une solution aqueuse de soude 5N puis 100 cm3 d'eau distillée. La solution organique est décantée et la phase aqueuse est lavée avec 2 fois 50 cm3 d'acétate d'éthyle. Les extraits organiques sont réunis, séchés sur sulfate de magnésium anhydre, filtrés et évaporés à sec sous pression réduite (20 mm de mercure ; 2,7 kPa) à 40°C. Le résidu est dissous dans 75 cm3 de propanol-2 ; on ajoute 0,5 g de noir décolorant et chauffe le mélange à 100°C pendant 30 minutes, filtre sur terre de diatomée et refroidit

à 10°C pendant 1 heure. Le précipité formé est séparé par filtration, lavé par 2 fois 10 cm3 de propanol-2. On obtient ainsi 8 g de [(méthyl-4 benzyl)-4 pipérazinyl-1]-2 phényl-4 pyridine fondant à 103°C.

La chloro-2 phényl-4 pyridine peut être préparée de la manière suivante : Un mélange de 26 g de chlorhydrate de N-oxyde de phényl-4 pyridine et de 75 cm3 de chlorure de phosphoryle est chauffé pendant 16 heures à 190°C dans un autoclave recouvert intérieurement d'une enveloppe en tantale. Après refroidissement à une température voisine de 20°C, l'excès de chlorure de phosphoryle est éliminé par distillation sous pression réduite (20 mm de mercure ; 2,7 kPa) à 50°C. Le résidu est repris par 100 cm3 d'eau glacée, 40 cm3 de solution aqueuse de soude 5N. La solution aqueuse est lavée avec 1 fois 500 cm3 puis avec 2 fois 250 cm3 d'acétate d'éthyle. Les extraits organiques sont réunis, séchés sur sulfate de magnésium anhydre, filtrés et concentrés à sec sous pression réduite (20 mm de mercure ; 2,7 kPa) à 40°C. Le résidu obtenu est dissous dans 40 cm3 de chlorure de méthylène et la solution est versée sur 500 g de silice contenus dans une colonne de 6 cm de diamètre. On élue avec 3 litres de chlorure de méthylène et l'éluat est évaporé à sec sous pression réduite (20 mm de mercure ; 2,7 kPa) à 40°C. On obtient ainsi 18,1 g de chloro-2 phényl-4 pyridine fondant à 70-72°C.

Le N-oxyde de phényl-4 pyridine peut être préparé selon la méthode décrite par K. KAWEMATSU, M. TAKEDA, A.E. JACOBSON et E.L. MAY, J. Med. Chem., 12, 405 (1969).

EXEMPLE 4

A une solution agitée de 5,4 g de phényl-3 (pyridyl-2)-3 propènal dans 150 cm3 de tétrahydrofuranne, on ajoute en 15 minutes à une température voisine de 20°C une solution de 14 g de (méthyl-4 benzyl)-1 pipérazine dans 30 cm3 de tétrahydrofuranne et on laisse agiter pendant 8 heures à une température voisine de 66°C. Après refroidissement de la solution à une température voisine de 20°C, le mélange réactionnel est évaporé à sec sous pression réduite (20 mm de

mercure ; 2,7 kPa) à 40°C. Le résidu obtenu est dissous dans 50 cm3 d'éther éthylique et la solution obtenue est versée sur 400 g de silice contenus dans une colonne de 4,7 cm de diamètre. On élue avec 300 cm3 d'un mélange d'éther éthylique et d'éther de pétrole (40-65°C) (80-20 en volumes) et l'éluat correspondant est concentré à sec sous pression réduite (20 mm de mercure ; 2,7 kPa) à 40°C. L'huile obtenue est mise en solution dans 200 cm3 d'éther éthylique bouillant. On ajoute 3 cm3 d'une solution bouillante 2,5N d'acide chlorhydrique dans l'alcool éthylique. Après refroidissement à une température voisine de 20°C, le précipité formé est séparé par filtration, puis recristallisé dans 400 cm3 d'alcool éthylique bouillant. On obtient ainsi 3,1 g de chlorhydrate de [(méthyl-4 benzyl)-4 pipérazinyl-1]-3 phényl-1 indolizine fondant à 210°C avec décomposition.

Le phényl-3 (pyridyl-2)-3 propènal peut être préparé de la façon suivante : A une suspension agitée sous atmosphère d'azote de 3 g d'hydrure de sodium (préalablement lavé avec 2 fois 20 cm3 d'éther éthylique sec) et de 40 cm3 de tétrahydrofuranne sec, on ajoute en 15 minutes à une température voisine de 20°C une solution de 17 g de (cyclohexylamino-2 vinyl)-1 phosphonate de diéthyle dans 300 cm3 de tétrahydrofuranne sec et on laisse agiter pendant 2 heures à une température voisine de 45°C. Après refroidissement de la solution à une température voisine de 20°C, on ajoute 8 g de benzoyl-2 pyridine en solution dans 100 cm3 de tétrahydrofuranne sec et on laisse agiter pendant 5 heures à une température voisine de 45°C. Après refroidissement de la solution à une température voisine de 20°C, on ajoute 10 cm3 d'eau distillée et on évapore sous pression réduite (20 mm de mercure ; 2,7 kPa) à 40°C le tétrahydrofuranne. Au résidu obtenu, on ajoute 20 g d'acétate de sodium dissous dans un mélange de 12 g d'acide acétique et 200 cm3 d'eau distillée, puis 400 cm3 d'éther éthylique et agite pendant 20 minutes. La phase éthérée est décantée et la phase aqueuse est extraite avec 2 fois 200 cm3 d'éther éthylique. Les phases organiques sont réunies, lavées avec 100 cm3 d'une solution aqueuse saturée de bicarbonate de sodium, puis avec 100 cm3 d'une solution aqueuse saturée de chlorure de

sodium, séchées sur sulfate de magnésium, filtrées et évaporées sous pression réduite (20 mm de mercure ; 2,7 kPa) à 40°C. Le résidu est dissous dans 50 cm3 d'éther éthylique et la solution est versée sur 800 g de silice contenus dans une colonne de 6,5 cm de diamètre. On élue d'abord avec 500 cm3 d'un mélange d'éther éthylique et d'éther de pétrole (40-65°C) (35-65 en volumes) ; les éluats correspondants sont éliminés. On élue ensuite avec 500 cm3 d'un mélange d'éther éthylique et d'éther de pétrole (40-65°C) (50-50 en volumes) et l'éluat correspondant est concentré à sec sous pression réduite (20 mm de mercure ; 2,7 kPa) à 40°C. On obtient ainsi 5,4 g de phényl-3 (pyridyl-2)-3 propènal utilisé à l'état brut dans les synthèses ultérieures.

Le (cyclohexylamino-2 vinyl)-1 phosphonate de diéthyle peut être préparé selon la méthode décrite par V. NAGATA et Y. HAYASE, J. Chem. Soc. (C), 460 (1969).

EXEMPLE 5

A une solution agitée de 1 g de phényl-3 pyrazinyl-3 propènal dans 50 cm3 de tétrahydrofuranne, on ajoute en 10 minutes à une température voisine de 20°C une solution de 9,5 g de (méthyl-4 benzyl)-1 pipérazine dans 50 cm3 de têtrahydrofuranne et on laisse agiter pendant 3 heures à une température voisine de 66°C. Après refroidissement de la solution à une température voisine de 20°C, le mélange réactionnel est concentré à sec sous pression réduite (20 mm de mercure ; 2,7 kPa) à 40°C. On obtient un résidu que l'on dissout dans 10 cm3 de chlorure de méthylène. La solution obtenue est versée sur 200 g de silice contenus dans une colonne de 3,5 cm de diamètre. On élue avec 500 cm3 d'un mélange de chlorure de méthylène et de méthanol (99,5-0,5 en volumes) et l'éluat correspondant est évaporé à sec sous pression réduite (20 mm de mercure ; 2,7 kPa) à 40°C. L'huile obtenue est dissoute dans 20 cm3 de méthyléthylcétone bouillante. On ajoute une solution de 0,25 g d'acide oxalique dans 20 cm3 de méthyléthylcétone bouillante. Après refroidissement à une température voisine de 20°C le précipité formé est séparé par filtration,

puis recristallisé dans 80 cm3 d'alcool isopropylique bouillant. On obtient ainsi 0,4 g d'oxalate de [(méthyl-4 benzyl)-4 pipérazinyl-1]-3 phényl-1 pyrrolo[1,2-a] pyrazine fondant à 128°C.

Le phényl-3 pyrazinyl-3 propènal peut être préparé de la manière suivante : A une suspension agitée sous atmosphère d'azote de 4,1 g d'hydrure de sodium (préalablement lavé avec 2 fois 30 cm3 d'éther éthylique sec) et de 50 cm3 de tétrahydrofuranne sec, on ajoute en 30 minutes à une température de 20°C une solution de 44,3 g de (cyclohexylamino-2 vinyl)-1 phosphonate de diéthyle dans 300 cm3 de tétrahydrofuranne sec et on laisse agiter pendant 2 heures à une température voisine de 45°C. Après refroidissement de la solution à une température voisine de 20°C, on ajoute 15,6 g de benzoyl pyrazine en solution dans 200 cm3 de tétrahydrofuranne sec et on laisse agiter pendant 5 heures à une température voisine de 45°C. Après refroidissement de la solution à une température voisine de 20°C, on ajoute 15 cm3 d'eau distillée et on évapore à sec sous pression réduite (20 mm de mercure ; 2,7 kPa) à 40°C. On obtient un résidu que l'on dissout dans 150 cm3 de chlorure de méthylène. La solution est versée sur 700 g de silice contenus dans une colonne de 8 cm de diamètre. On élue avec 1200 cm3 de chlorure de méthylène ; l'éluat correspondant est concentré à sec sous pression réduite (20 mm de mercure ; 2,7 kPa) à 40°C. On obtient ainsi 1 g de phényl-3 pyrazinyl-3 propènal sous forme d'une huile brune. Rf = 0,58 [chromatographie sur couche mince de gel de silice ; solvant : chlorure de méthylène-méthanol (95-5)].

La benzoyl pyrazine peut être préparée de la façon suivante : A 4,1 g de magnésium en tournures, on ajoute en 1 heure en agitant sous atmosphère d'azote, à une température voisine de 20°C, une solution de 32,5 g de bromobenzène dans 350 cm3 d'éther éthylique et on laisse agiter pendant encore 1 heure à une température voisine de 35°C puis pendant 15 heures à une température voisine de 20°C. On ajoute ensuite 17,4 g de cyanopyrazine dans 150 cm3 d'éther éthylique et laisse agiter pendant 2 heures et demie. On ajoute alors 250 cm3 d'eau distillée, laisse agiter pendant 1 heure et demie à une température voisine de 5°C, ajoute 150 cm3 d'une solution aqueuse saturée

de chlorure d'ammonium et laisse agiter pendant 1 heure. On décante la phase éthérée et extrait la phase aqueuse avec 3 fois 100 cm3 d'éther éthylique. Les phases organiques sont réunies, lavées avec 20 cm3 d'eau distillée, séchées sur sulfate de magnésium et filtrées. Le solvant est évaporé sous pression réduite (20 mm de mercure ; 2,7 kPa) à 30°C. Le résidu obtenu est dissous dans 100 cm3 de cyclohexane et la solution obtenue est versée sur 600 g de silice contenus dans une colonne de 5 cm de diamètre. On élue avec 1 litre d'un mélange de cyclohexane et d'acétate d'éthyle (95-5 en volumes) ; les éluats correspondants sont éliminés. On élue ensuite avec 4 litres du même mélange et l'éluat correspondant est évaporé à sec sous pression réduite (20 mm de mercure ; 2,7 kPa) à 40°C. On obtient ainsi 12,5 g de benzoyl pyrazine sous forme d'une huile orange. Rf = 0,35 [chromatographie sur couche mince de gel de silice ; solvant : cyclohexane-acétate d'éthyle (60-40)].

La cyanopyrazine peut être préparée suivant la méthode décrite par M. ROBBA, Ann. de Chimie, 13 (B) 379 (1960).

Le (cyclohexylamino-2 vinyl)-1 phosphonate de diéthyle peut être préparé selon la méthode décrite par V. NAGATA et Y. HAYASE, J. Chem. Soc. (C) 460 (1969).

EXEMPLE 6

A 11,2 g de (méthyl-4 benzyl)-4 pipérazine on ajoute une solution de 1,25 g de phényl-3 (pyrimidinyl-2)-3 propènal dans 125 cm3 de tétrahydrofuranne. La solution est agitée et chauffée à une température voisine de 65°C pendant 3 heures. Après refroidissement à une température voisine de 20°C, le mélange réactionnel est concentré à sec sous pression réduite (20 mm de mercure ; 2,7 kPa) à 40°C. Le résidu obtenu est dissous dans 10 cm3 d'acétate d'éthyle et la solution obtenue est versée sur 25 g de silice contenus dans une colonne de 1,5 cm de diamètre. On élue avec 750 cm3 d'un mélange d'acétate d'éthyle et de cyclohexane (50-50 en volumes) ; les éluats correspondants sont éliminés. On élue ensuite avec 300 cm3 du même mélange et l'éluat correspondant est concentré à sec sous pression

réduite (20 mm de mercure ; 2,7 kPa) à 40°C. L'huile obtenue est mise en solution dans 20 cm3 de méthyléthylcétone. On ajoute 120 mg d'acide oxalique dissous dans 20 cm3 de méthyléthylcétone et agite le mélange pendant environ 15 minutes à une température voisine de 20°C. Le précipité formé est séparé par filtration, puis recristallisé dans 50 cm3 d'alcool isopropylique bouillant. On obtient ainsi 300 mg d'oxalate de [(méthyl-4 benzyl)-4 pipérazinyl-1)]-3 phényl-1 pyrrolo[1,2-a] pyrimidine fondant à 205°C.

Le phényl-3 (pyrimidinyl-2)-3 propènal peut être préparé de la manière suivante : On agite fortement une suspension de 1,5 g de phényl-3 (pyrimidinyl-2)-3 propèn-2 ol-1 et de 6,25 g de bioxyde de manganèse dans 125 cm3 de dioxanne à une température voisine de 20°C pendant environ 16 heures. Le précipité est ensuite séparé par filtration et le filtrat est évaporé à sec sous pression réduite (20 mm de mercure ; 2,7 kPa) à 60°C. On obtient ainsi 1,25 g de phényl-3 (pyrimidinyl-2)-3 propènal sous forme d'une huile jaune. Rf = 0,78 (chromatographie sur couche mince de gel de silice ; solvant : acétate d'éthyle).

Le phényl-3 (pyrimidinyl-2)-3 propèn-2 ol-1 peut être préparé de la façon suivante : A une solution de 2,5 g de phényl-3 (pyrimidinyl-2)-3 acrylate de butyle dans 150 cm3 de toluène sec maintenue sous atmosphère d'azote à une température voisine de -78°C, on ajoute en 15 minutes environ 15 cm3 d'une solution 1,2M d'hydrure de diisobutylaluminium dans le toluène et poursuit l'agitation pendant 1 heure à une température voisine de -78°C. On laisse ensuite remonter la température à environ 0°C pendant environ 3 heures puis on ajoute 150 cm3 d'une solution aqueuse saturée de chlorure d'ammonium, décante et sépare la phase organique. La phase aqueuse est extraite 2 fois avec 150 cm3 au total de chlorure de méthylène. Les phases organiques sont réunies et séchées sur sulfate de magnésium, filtrées et concentrées à sec sous pression réduite (20 mm de mercure ; 2,7 kPa) à 50°C. On obtient ainsi 1,5 g de phényl-3 (pyrimidinyl-2)-3 propèn-2 ol-1 sous forme d'une huile jaune. Rf = 0,57 (chromatographie sur couche mince de gel de silice ; solvant : acétate d'éthyle).

Le phényl-3 (pyrimidinyl-2)-3 acrylate de butyle peut être préparé de la manière suivante : A une suspension de 12,2 g de bromure de tributylbenzylphosphonium dans 140 cm3 de diméthoxy-1,2 éthane sec agitée sous atmosphère d'azote à une température voisine de -30°C, on ajoute en 20 minutes environ 20 cm3 d'une solution 1,6M de n-butyllithium dans l'hexane. On laisse la suspension remonter à une température voisine de 20°C en 1 heure environ et poursuit l'agitation pendant encore 1 heure à cette même température. On ajoute ensuite 1,75 g de chloro-2 pyrimidine en suspension dans 40 cm3 de diméthoxyéthane sec et chauffe pendant environ 5 heures à une température voisine de 70°C. La température de la solution est ensuite abaissée à environ 20°C. On ajoute alors 10 cm3 d'une solution de n-butyllithium 1,6M dans l'hexane et chauffe à nouveau la suspension à 70°C environ pendant 3 heures. Après refroidissement de la solution à une température voisine de 20°C, le mélange réactionnel est ajouté en 30 minutes à une solution de 3,8 g de glyoxylate de butyle dans 20 cm3 de diméthoxy-1,2 éthane et la suspension est chauffée pendant 24 heures à une température voisine de 70°C. Après refroidissement de la solution à une température voisine de 20°C, la suspension obtenue est filtrée. Le filtrat est dilué par 200 cm3 d'éther éthylique et lavé avec 200 cm3 d'eau distillée. On décante, sépare la phase organique et extrait la phase aqueuse avec 100 cm3 d'éther éthylique. Les phases organiques sont réunies, séchées sur sulfate de magnésium, filtrées et concentrées à sec sous pression réduite (20 mm de mercure ; 2,7 kPa) à 30°C. Le résidu obtenu est dissous dans 30 cm3 de chlorure de méthylène et la solution est versée sur 500 g de silice contenus dans une colonne de 5 cm de diamètre. On élue avec 1000 cm3 d'un mélange de chlorure de méthylène et de cyclohexane (50-50 en volumes) ; les éluats correspondants sont éliminés. On élue ensuite avec 500 cm3 du même mélange et l'éluat correspondant est évaporé à sec sous pression réduite (20 mm de mercure ; 2,7 kPa) à 40°C. On obtient ainsi 2,5 g de phényl-3 (pyrimidinyl-2)-3 acrylate de butyle sous forme d'une huile jaune. Rf = 0,75 (chromatographie sur couche mince de gel de silice ; solvant : acétate d'éthyle).

La présente invention concerne également les médicaments constitués par un produit de formule générale (I), sous forme libre ou sous forme de sel d'addition avec un acide ou une base pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale ou parentérale.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en

incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

En thérapeutique humaine, les produits selon l'invention sont particulièrement utiles dans le traitement des troubles du psychisme et plus particulièrement des psychoses telles que la schizophrénie ou les délires. Les doses dépendant de l'effet recherché et de la durée du traitement ; elles sont généralement comprises entre 25 et 250 mg par jour par voie orale pour un adulte en une ou plusieurs prises.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

EXEMPLE A

On prépare, selon la technique habituelle, des comprimés dosés à 25 mg de produit actif ayant la composition suivante :

- [(méthyl-4 benzyl)-4 pipérazinyl-1]-3 biphényle, dichlorhydrate .................................... 30,25 mg
- amidon .......................................... 60 mg
- lactose ......................................... 50 mg
- stéarate de magnésium ............................ 2 mg

EXEMPLE B

On prépare une solution injectable contenant 25 mg de produit actif ayant la composition suivante :

- [(méthyl-4 benzyl)-4 pipérazinyl-1]-2 phényl-4 pyridine..................................... .... 25 mg
- solution aqueuse d'acide chlorhydrique 0,1N ....... 2,18 cm3
- soluté injectable .........................qsp 10 cm3

REVENDICATIONS

1 - Nouveau dérivé de la benzyl-4 pipérazine, caractérisé en ce qu'il répond à la formule générale :

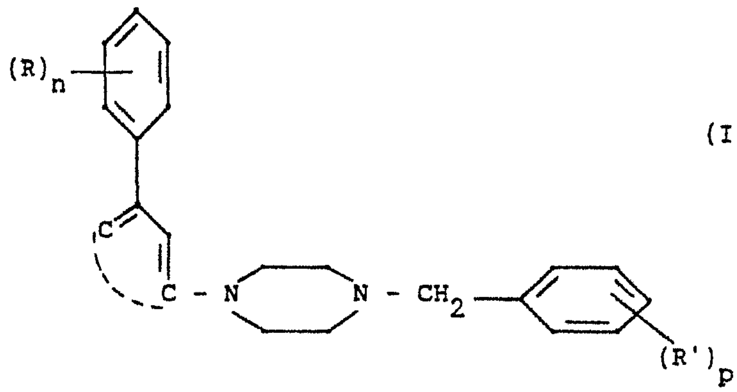

(I)

dans laquelle la ligne pointillée forme avec la chaîne de quatre atomes de carbone à laquelle elle se rattache un cycle aromatique choisi parmi les cycles phényle, naphtyle, pyridine, indolizine, pyrrolo[1,2-a] pyrazine, pyrrolo[1,2-a] pyrimidine ou pyrrolo[1,2-c] pyrimidine, R représente un atome d'hydrogène ou d'halogène ou un radical hydroxy, alcoyle, alcoyloxy ou alcoylthio, R' représente un atome d'hydrogène ou d'halogène ou un radical alcoyle, alcoyloxy, alcoylthio, cyano ou trifluorométhyle, n et p représentent les nombres entiers 1, 2 ou 3, étant entendu que les radicaux alcoyle et portions alcoyles contiennent 1 à 4 atomes de carbone en chaîne droite ou ramifiée, ainsi que ses sels d'addition avec les acides pharmaceutiquement acceptables.

2 - Procédé de préparation d'un produit selon la revendication 1 dans la formule duquel la ligne pointillée forme avec la chaîne de quatre atomes de carbone à laquelle elle se rattache un cycle phényle ou naphtyle, caractérisé en ce que l'on fait réagir un produit de formule générale :

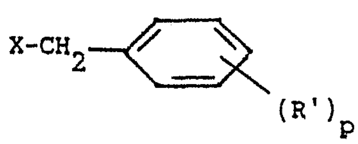

(II)

dans laquelle R' et p sont définis comme à la revendication 1 et X représente un atome d'halogène ou un reste d'ester activé sur un produit de formule générale :

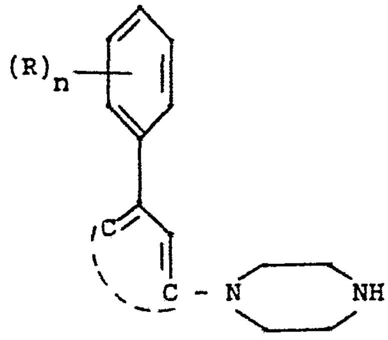

(III)

dans laquelle la ligne pointillée forme avec la chaîne de quatre atomes de carbone à laquelle elle se rattache un cycle phényle ou naphtyle et R et n sont définis comme à la revendication 1, puis isole le produit obtenu et le transforme éventuellement en un sel d'addition avec un acide pharmaceutiquement acceptable.

3 - Procédé de préparation d'un produit selon la revendication 1 dans la formule duquel la ligne pointillée forme avec la chaîne de quatre atomes de carbone à laquelle elle se rattache un cycle pyridine, caractérisé en ce que l'on fait réagir une pipérazine de formule générale :

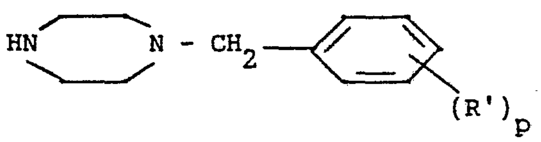

(V)

dans laquelle R' et p sont définis comme à la revendication 1, sur un produit de formule générale :

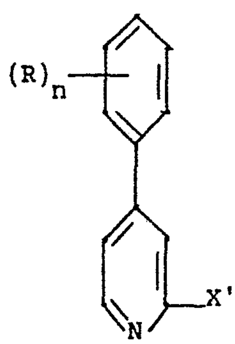

(VI)

dans laquelle R et n sont définis comme à la revendication 1 et X' représente un atome d'halogène, puis isole le produit obtenu et le transforme éventuellement en un sel d'addition avec un acide pharmaceutiquement acceptable.

4 - Procédé de préparation d'un produit selon la revendication 1 dans la formule duquel la ligne pointillée forme avec la chaîne de quatre atomes de carbone à laquelle elle se rattache un cycle indolizine, pyrrolo[1,2-a] pyrazine, pyrrolo[1,2-a] pyrimidine ou pyrrolo[1,2-c] pyrimidine, caractérisé en ce que l'on fait réagir une pipérazine de formule générale :

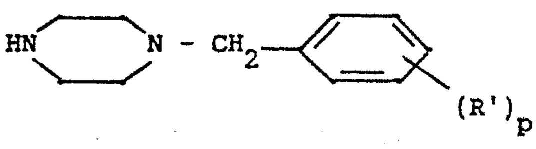

(V)

dans laquelle R' et p sont définis comme à la revendication 1 sur un aldéhyde de formule générale :

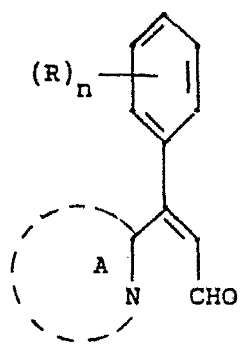

(VIII)

dans laquelle R et n sont définis comme à la revendication 1 et A représente un radical pyridyle-2, pyrazinyle, pyrimidinyle-2 ou pyrimidinyle-4, puis isole le produit obtenu et le transforme éventuellement en un sel d'addition avec un acide pharmaceutiquement acceptable.

5 - Composition pharmaceutique, caractérisée en ce qu'elle contient comme ingrédient actif au moins un produit selon la revendication 1 en association avec un ou plusieurs diluants ou adjuvants compatibles et pharmaceutiquement acceptables.

REVENDICATION

Procédé de préparation de nouveaux dérivés de la benzyl-4 pipérazine, caractérisé en ce qu'il répond à la formule générale :

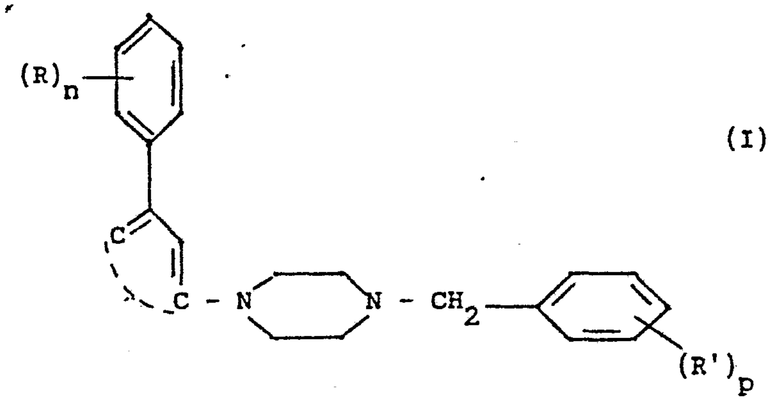

(I)

dans laquelle la ligne pointillée forme avec la chaîne de quatre atomes de carbone à laquelle elle se rattache un cycle aromatique choisi parmi les cycles phényle, naphtyle, pyridine, indolizine, pyrrolo[1,2-a] pyrazine, pyrrolo[1,2-a] pyrimidine ou pyrrolo[1,2-c] pyrimidine, R représente un atome d'hydrogène ou d'halogène ou un radical hydroxy, alcoyle, alcoyloxy ou alcoylthio, R' représente un atome d'hydrogène ou d'halogène ou un radical alcoyle, alcoyloxy, alcoylthio, cyano ou trifluorométhyle, n et p représentent les nombres entiers 1, 2 ou 3, étant entendu que les radicaux alcoyle et portions alcoyles contiennent 1 à 4 atomes de carbone en chaîne droite ou ramifiée, ainsi que leurs sels d'addition avec les acides pharmaceutiquement acceptables, caractérisé en ce que,

A - pour la préparation d'un produit de formule générale (I) dans laquelle la ligne pointillée forme avec la chaîne de quatre atomes de carbone à laquelle elle se rattache un cycle phényle ou naphtyle, on fait réagir un produit de formule générale :

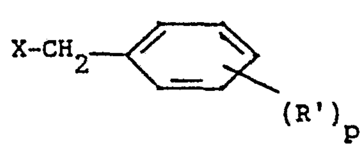

(II)

dans laquelle R' et p sont définis comme précédemment et X représente un atome d'halogène ou un reste d'ester activé sur un produit de formule générale :

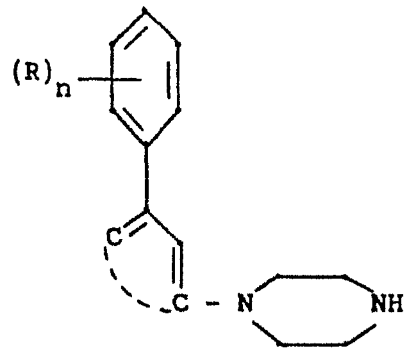

(III)

dans laquelle la ligne pointillée forme avec la chaîne de quatre atomes de carbone à laquelle elle se rattache un cycle phényle ou naphtyle et R et n sont définis comme précédemment, puis isole le produit obtenu et le transforme éventuellement en un sel d'addition avec un acide pharmaceutiquement acceptable, ou en ce que

B - pour la préparation d'un produit de formule générale (I) dans laquelle la ligne pointillée forme avec la chaîne de quatre atomes de carbone à laquelle elle se rattache un cycle pyridine, on fait réagir une pipérazine de formule générale :

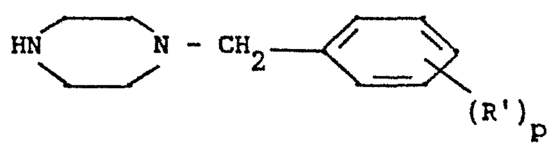

(V)

dans laquelle R' et p sont définis comme précédemment, sur un produit de formule générale :

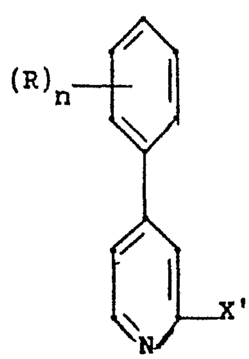

(VI)

dans laquelle R et n sont définis comme précédemment et X' représente un atome d'halogène, puis isole le produit obtenu et le transforme éventuellement en un sel d'addition avec un acide pharmaceutiquement acceptable, ou en ce que

C - pour la préparation d'un produit de formule générale (I) dans laquelle la ligne pointillée forme avec la chaîne de quatre atomes de carbone à laquelle elle se rattache un cycle indolizine, pyrrolo [1,2-a] pyrazine, pyrrolo[1,2-a] pyrimidine ou pyrrolo[1,2-c] pyrimidine, on fait réagir une pipérazine de formule générale :

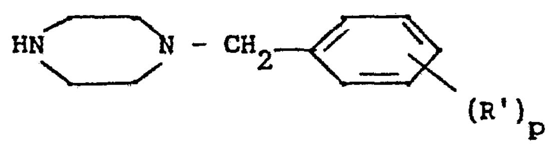

(V)

dans laquelle R' et p sont définis comme précédemment sur un aldéhyde de formule générale :

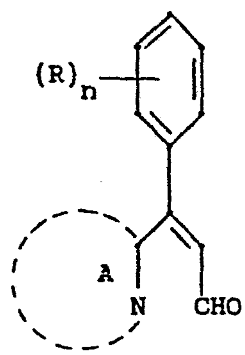

(VIII)

dans laquelle R et n sont définis comme précédemment et A représente un radical pyridyle-2, pyrazinyle, pyrimidinyle-2 ou pyrimidinyle-4, puis isole le produit obtenu et le transforme éventuellement en un sel d'addition avec un acide pharmaceutiquement acceptable.

Office européen des brevets

# RAPPORT DE RECHERCHE EUROPEENNE

0256936

Numero de la demande

EP 87 40 1834

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | CHEMICAL AND PHARMACEUTICAL BULLETIN, vol. 28, no. 9, 1980, pages 2618-2622, Tokyo, JP; K. HINO et al.: "4-Phenyl-2-(1-piperazinyl)quinolines with potent antidepressant activity" * Pages 2619,2620, composé 7h * | 1,5 | C 07 D 295/02<br>C 07 D 213/74<br>C 07 D 471/04<br>C 07 D 487/04<br>A 61 K 31/495//<br>(C 07 D 471/04<br>C 07 D 221:00<br>C 07 D 209:00 )<br>(C 07 D 487/04<br>C 07 D 241:00<br>C 07 D 209:00 )<br>(C 07 D 487/04<br>C 07 D 239:00<br>C 07 D 209:00 ) |
| Y | CHEMICAL ABSTRACTS, vol. 77, 1972, pages 496-497, résumé no. 48507n, Columbus, Ohio, US; & JP-A-72 17 306 (YOSHITOMI PHARMACEUTICAL INDUSTRIES, LTD) 20-05-1972 | 1,5 | |
| Y | FR-M- 2 533 (SIFA) * Résumé, point 1 * | 1,5 | |
| Y | EP-A-0 100 257 (DELALANDE) * Revendications 1,3 * | 1,5 | |

**DOMAINES TECHNIQUES RECHERCHES (Int. Cl.4)**

C 07 D 295/00
C 07 D 213/00
C 07 D 471/00
C 07 D 487/00
A 61 K 31/00

Le présent rapport a été établi pour toutes les revendications

| Lieu de la recherche | Date d'achèvement de la recherche | Examinateur |
|---|---|---|
| LA HAYE | 16-11-1987 | ALFARO I. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)